# EUROPEAN PATENT SPECIFICATION

(11) **EP 1 237 489 B1**
(45) Date of publication and mention of the grant of the patent: **08.02.2006**
(21) Application number: 00990474.9
(22) Date of filing: 01.11.2000
(51) Int. Cl.: A61B 17/22, A61B 17/34, A61B 1/00, A61B 1/307, A61B 1/313

(54) **ENDARTERECTOMY SURGICAL INSTRUMENT**
CHIRURGISCHES INSTRUMENT ZUR ARTERIENINNENHAUTENTFERNUNG
INSTRUMENT CHIRURGICAL POUR L'ENDARTERIECTOMIE

(30) Priority: 16.11.1999 US 165707 P
(43) Date of publication of application: 11.09.2002
(73) Proprietor: DEKA PRODUCTS LIMITED PARTNERSHIP, Manchester, NH 03101 (US)
(72) Inventor: KAMEN, Dean, L., Bedford, NH 03110 (US); GRANT, Kevin, Lee, Litchfield, NH 03052 (US); GRAY, Larry, B., Merrimack, NH 03054 (US); GRINNELL, Charles, M., Groton, MA 01450 (US); KAPLITT, Martin, J., Floral Park, NY 11005 (US); NEWMAN, Fredric, A., Scarsdale, NY 10583 (US); TRACEY, Brian, Daniel, Litchfield, NH 03052 (US)
(74) Representative: Greene, Simon Kenneth
(86) International application number: PCT/US2000/041752
(87) International publication number: WO 2001/035840

(56) References cited:
- US-A- 4 557 255
- US-A- 4 759 348
- US-A- 5 364 365
- US-A- 5 891 013
- US-A- 5 954 713

## Description

### Field of the Invention

The present invention pertains to surgical instruments and procedures, and more particularly to the removal of plaque build-ups within blood vessels.

### Background Art

Excessive plaque build-up within arteries decreases the blood flow capacity of the arteries and of the living tissue supplied by the arteries. Normal blood flow may be restored by either removing the plaque build-up or by bypassing the blocked section of the artery. In a bypass procedure, a second blood vessel is attached to, and parallel to, the vessel to be bypassed and provides a flow path around the blocked section of the artery. The second blood vessel has one end attached upstream of the blocked section and the second end attached downstream of the blocked section. Alternatively, the blockage may be removed from the artery by opening the artery along the blockage and removing the blockage. Both procedures require incisions along the blockage and become extremely invasive for extensive blockages that may run the length from the groin to the knee, for example.

A less invasive procedure for removing plaque build-ups requires only two incision points; one above the blockage and one below the blockage. A catheter is inserted into the artery at the up-stream incision point and is pushed toward the second incision point that is downstream from the blockage. The catheter head is configured to either grab or loosen the plaque build-up along the blockage. As the catheter is passed through the blockage, the build-up is pushed ahead of the catheter and is removed at the second incision point. Although the catheter will remove sufficient blockage to restore normal flow, the procedure may not remove all the build-up. Depending on the type of catheter head used in the procedure, there exists a risk that the catheter head may penetrate through the plaque build-up layer, the intima and media layers of the artery and damage the adventitia layer of the artery. In addition, the procedure does not remove the blockage from the side branches of the artery and may worsen the blockage of the side branches by a "snowplow" effect.

Instead of pushing a catheter through the blood flow channel of the artery, gas endarterectomy uses a gas to separate the media layer surrounding the blockage from the adventitia layer of the artery. Once separated from the adventitia layer of the artery, the blockage may be easily removed from the downstream incision point or from the initial incision point if the blockage is less than about an inch. The downstream incision point is still required because the intima and media layers of the artery attached to the blockage site must be separated from the intima and media layers of the non-blocked artery section. Although the gas endarterectomy procedure also requires two incisions, the procedure does not suffer from the "snowplow" effect of the catheter procedure and may also remove the side branch plugs along with the main blockage. In addition, all plaque is removed in a gas endarterectomy procedure because the underlying intima and media layers containing the plaque are removed.

In U.S. patent number 5,954,713 ('713) issued on September 21,1999 to Newman, et. al., a gas endarterectomy procedure is described wherein only one incision point is required. The '713 patent describes a spatula head having an optical channel for illumination and viewing of the space in front of the spatula head, gas flow channels for the gas that separates the media layer from the adventitia layer, and liquid flow channels for clearing the optics and removing debris. In the procedure described in the '713 patent, after the spatula head is used to separate the blockage from the artery, the spatula head is removed from the artery and a surgical cutting instrument is inserted into the artery and moved to the end of the blockage whereupon the surgical cutting instrument cuts the intima and media layers of the artery. The blockage is then removed from the artery by pulling the blockage through the incision using forceps or the surgical cutting instrument.

As described, however, the procedure described in the'713 patent requires two instruments: the spatula instrument and the cutting instrument. Furthermore, both instruments must be worked through the blockage. This increases the duration of the requisite operation and the risk of damage to the adventitia layer of the artery. It is thus preferable to have a single instrument that both separates the media layer from the adventitia layer and then removes the blockage.

The media and intima layers are preferably cut at the point where the gas endarterectomy has separated the layers from the adventitia layer. Using prior art practice, damage to the adventitia may occur if the cut is made beyond the separation point. If the cut is made behind the separation point, the separated media and intima layers will form a flap within the artery. In accordance with prior art practice, a stent is usually inserted to prevent formation of the flap. The stent covers the transition region between the section of the artery where the media and intima layers have been removed with the blockage and the section of the artery where the media and intima layers are intact.

US 4,557,255 describes a urteroscope with a telescope assembly and remotely operable jaws for retrieving stones and the like from the ureter.

US 5,364,365 describes a thin bodied surgical instrument with an obturator spring loaded within a cannula, together with a handle at the opposite end of the cannula.

### Summary of Invention

The invention relates to an endarterectomy surgical instrument as set out in claim 1.

It has been observed that the media and intima layer is fairly weak at the transition between the plaque build-up and no-plaque build-up regions. Therefore, by grabbing the blockage and pulling, the blockage will tend to separate from the healthy media and intima layers at the transition without the use of a cutting tool. By eliminating the cutting tool, risk to the patient may be advantageously reduced, additionally, only the diseased portion of the media and intima layers need be removed. Furthermore, in accordance with embodiments of the invention, the break occurs at the point where the healthy media and intima layers are separated, thereby producing a smoother transition region.

In accordance with embodiments of the present invention, a separate surgical cutting instrument may be eliminated by adding a grabbing or grasping function to the spatula head of the endarterectomy surgical instrument provided in accordance with the present invention. The spatula head contains either hooks, barbs, or prongs that can be deployed by the operator. The hooks or barbs may be retracted after deployment. A gas channel is preferably incorporated into the shaft of the instrument, either as a separate channel or with an endoscope in an optical channel.

The endarterectomy surgical instrument provided in accordance with preferred embodiments of the present invention has a shaft with proximal and distal ends. A head, coupled to the distal end of the shaft, has an endoscope port and at least one fluid port, while a handle, coupled to the proximal end of the shaft, has a gas supply port in fluid communication with the at least one gas port on the head, a flow valve for metering flow of gas between the gas supply port and the at least one fluid port on the head, and a locking mechanism for retaining an endoscope.

In accordance with alternate embodiments of the invention, the endarterectomy surgical instrument may also have saline solution inlet coupled to the handle for coupling a flow of saline solution to the at least one fluid port on the head. Fluid connection of the handle to the head of the shaft may be provided through a first lumen, while an endoscope may provide optical coupling through a second lumen between the distal and proximal ends of the shaft. The first and second lumens may be separate or identical.

The instrument also has a grasping device with both a retracted configuration and a deployed configuration, the grasping device extending away from the head in the deployed configuration. The grasping device may be a barb or a hook, and it may be controlled by a deployment control disposed on the handle of the instrument.

### Brief Description of the Drawings

FIG. 1a shows a perspective view of one embodiment of the present invention in the retracted configuration;
FIG. 1b shows a perspective view of the embodiment shown in FIG 1a in the deployed configuration;
FIG. 2a shows a front view of the head of the embodiment shown in FIG. 1a;
FIG. 2b shows a front view of the head of the embodiment shown in FIG. 1b;
FIG. 3a shows a front view of the head of the embodiment shown in FIG. 1a inside an artery;
FIG. 3b shows a front view of the head of the embodiment shown in FIG. 1b inside an artery;
FIG. 4a shows a perspective view of the head in another embodiment of the invention in the retracted configuration;
FIG. 4b shows a perspective view of the head of the embodiment shown in FIG 4a in the deployed configuration;
FIG.5a shows a front view of the head inside an artery of the embodiment shown in FIG. 4a;
FIG. 5b shows a front view of the head inside an artery of the embodiment shown in FIG. 4b;
FIG. 6 shows a cut side view of the handle of the embodiment of Fig. 1a;
FIG. 7a shows a cut bottom view of the head of the embodiment shown in FIG. 1a; and
FIG. 7b shows a cut bottom view of the head of the embodiment shown in FIG 1a in the deployed configuration;
FIG. 8 is a perspective view of the head of another embodiment with one of the hooks not shown; and
FIG. 9 shows a cut side view of the handle of the embodiment of Fig. 8.

### Detailed Description of Specific Embodiments

FIG. 1a shows a perspective view of an embodiment of the present invention in the retracted configuration. The gas endarterectomy surgical instrument, generally designated by numeral **10** comprises a head **11,** connected to a shaft **12,** which in turn is connected to a handle **13.** The handle **13** houses a gas port **14** that connects to a gas supply, not shown. The gas flow delivered to the head **11** is controlled by a variable flow valve **15.** An endoscope latch **16** is provided on the handle **13** to secure an endoscope to the surgical instrument **10.** The handle also houses a deployment control that controls the deployment or retraction of a grasping device located in the head **11.** In the embodiment shown in FIG. 1a, the deployment control is a slide **17.** The handle **13** is connected to shaft **12** which is a flexible tube providing multiple lumens as now described.

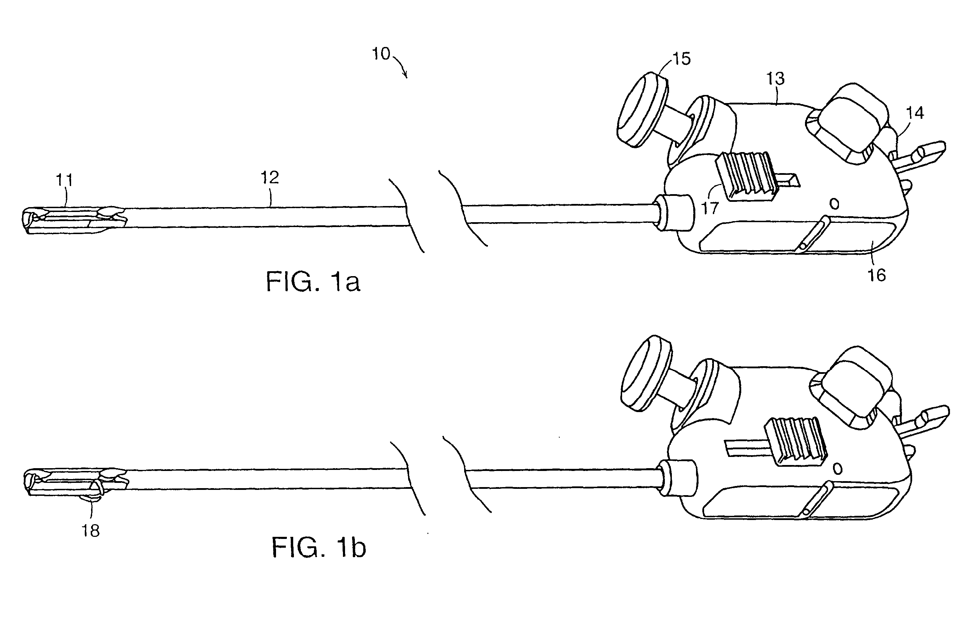

In accordance with this embodiment of the invention, shaft **12** is sized to provide flow paths both for the gas from the gas supply and for saline solution to the head **11** while also accommodating the endoscope and a control wire, not shown. The control wire is attached to the slide **17** on the handle at one end and is attached to the grasping device located in the head **11** at the other end. The head **11** is attached to the end of the shaft **12** opposite the end attached to the handle **13.** The head **11** is provided with an opening that holds the end of the endoscope and also provides exit orifices for the gas and saline solution. The head 11 also contains a grasping device that may be deployed by the operation of the deployment control. FIG. 1b shows the surgical instrument of FIG. 1a in the deployed configuration. In FIG. 1b, the slide **17** has been moved to the rear position, thereby deploying the grasping mechanism, which, in this embodiment of the invention, is a pair of barbs **18.**

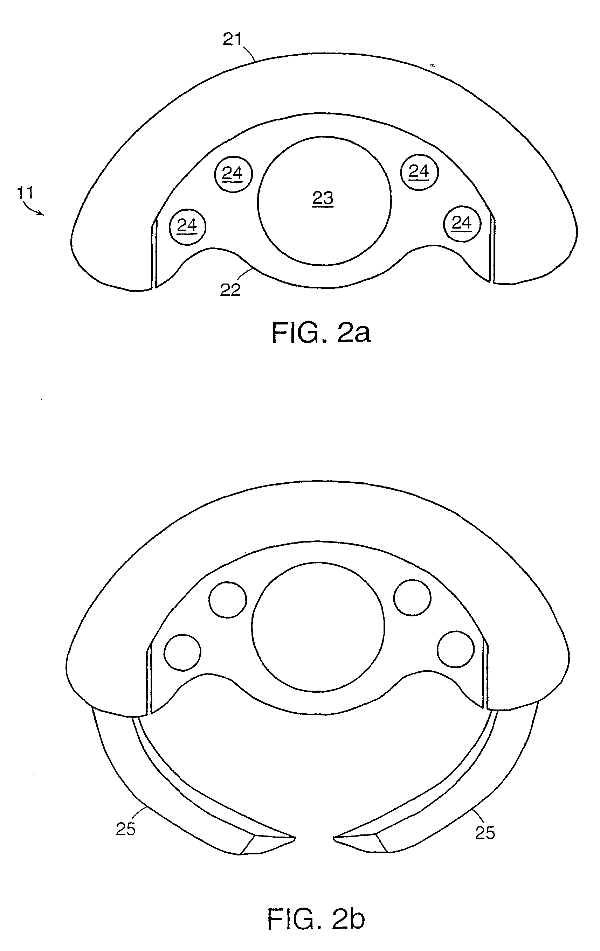

FIG. 2a shows a front view of head **11** in the retracted configuration of one embodiment of the invention. FIG. 2b shows a front view of head **11** in the deployed configuration of the embodiment shown in FIG. 2a. Head **11** has an outer side **21** that is shaped to approximate the inner wall of an artery. The inner side **22** of head **11** is generally flattened. The tip of head **11** contains an endoscope port **23** and a plurality of ports **24** for the delivery of gas and/or saline solution. The gas ports are in fluid communication with the gas flow path of the shaft **12** through internal channels within the head **11.** The end of the endoscope is held by the head by sizing the diameter of the endoscope port **23** to the size of the endoscope. FIG. 2b shows the front view of the head **11** in the deployed configuration. In the embodiment of the invention shown in FIG. 2b, a plurality of barbs or prongs **25** are deployed from the inner surface **22** and extend away and towards the rear of head **11.**

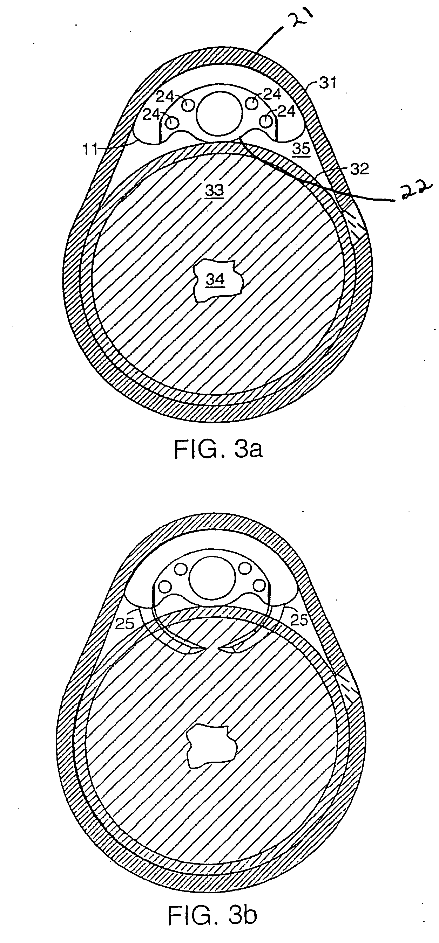

FIG. 3a shows a front view of head **11** of one embodiment of the invention placed in the artery of a patient. The head **11** of the surgical instrument is pushed between the adventitia layer **31** of the artery and the media layer **32** of the artery with the outer side **21** of the head **11** against the adventitia layer **31** of the artery and the inner side **22** of the head **11** facing the media layer **32** of the artery. The media layer **32** encircles the intima layer, not shown, on which the plaque forms a build-up layer **33** that obstructs the blood flow channel **34.** Gas jets from the gas ports **24** separates the adventitia layer **31** from the media layer **32,** creating an interstitial chamber **35** through which the head **11** can travel along the blockage caused by the plaque build-up layer **33.** FIG. 3b shows the front view of the head **11** shown in FIG. 3a with the barbs **25** deployed. The barbs **25** are deployed away from the inner surface **22** of the head **11** and extend into the media layer **32** and the build-up layer **33** of the obstruction. As the head **11** is pulled backward, the rear facing barbs **25** securely grab the obstruction, thereby removing the obstruction as the head **11** is removed from the artery. The barbs 25 may also be retracted after deployment by operating the deployment control to retract the barbs 25 while slightly urging the head **11** forward.

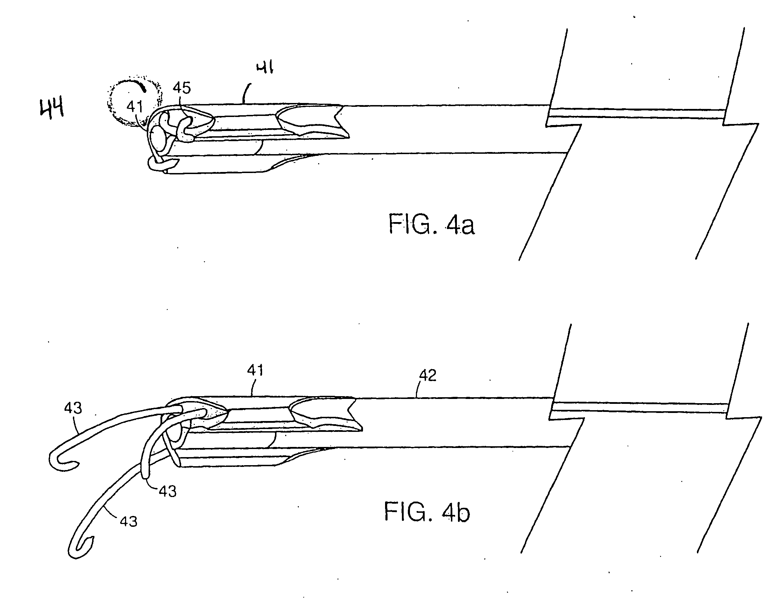

FIG. 4a shows a perspective view of the head of another embodiment of the present invention. FIG. 4b shows a perspective view of the head of the embodiment of the invention shown in FIG. 4a with the grasping mechanism deployed. In this embodiment of the invention, the grasping mechanism comprise a plurality of hooks 43. Hooks 43 are positioned at the front of the head **41** next to the endoscope port **44**. Hooks **43** are housed in channels **45** that terminate at the front of the head. The hooks **43** are attached to a control wire, not shown, that is passed through the shaft **42** and connected at the other end to the deployment control, not shown, on the handle.

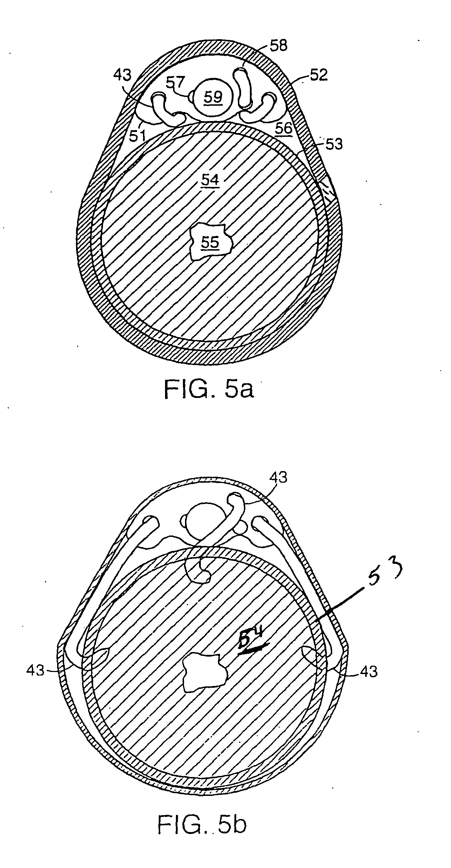

FIG. 5a shows a front view of the embodiment shown in FIG. 4a in the artery. FIG. 5b shows a front view of the embodiment shown in FIG. 4a in the artery of a patient in the deployed configuration. The head **51** is positioned between the adventitia layer **52** and the media layer **53** of the artery. Gas from gas ports **58** is used to open an interstitial space **56** between the adventitia layer **52** and the media layer **53** to allow the head **51** to travel parallel to the build-up layer **54** and the obstructed blood flow channel **55.** Each hook **43** is housed in a gas flow channel 58. Additional gas flow channels may be provided by side channels **57** on the endoscope port **59.** In the deployed configuration, as shown in FIG. 5b, the each hook **43** extends forward of the head **51.** As the head **51** is removed from the artery, each hook **43** grabs onto the media layer 53 and build-up layer **54** thereby removing the obstruction as the head **51** is removed from the artery.

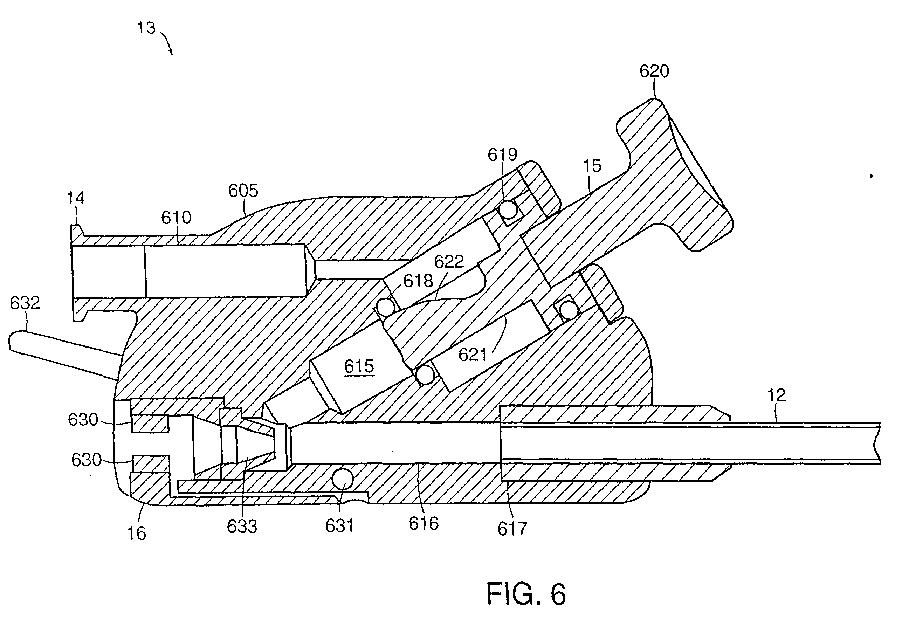

FIG. 6 is a side cut view showing the internal channels of the handle in one embodiment of the present invention. An external gas source is connected to the surgical instrument by a gas port **14** and supplies gas to the head of the surgical instrument. In a preferred embodiment, the gas is CO₂. The gas flows through the inlet passageway **610** to a variable flow valve **15,** exiting through an outlet passageway **615.** Outlet passageway **615** is connected to an endoscope passageway 616 which, in turn, is connected to the shaft **12** at the shaft end **617.**

Variable flow valve **15** comprises a knob **620** attached to a control piston **621,** the end of which may be displaced into or out of the outlet passageway **615.** The inlet passageway **610** is isolated from the outlet passageway **615** by a first sealing ring **618.** The inlet passageway **610** is maintained in fluid isolation with the environment by a second sealing ring **619.** The first sealing ring **618** is held against the handle body **605** by the control piston **621.** Control piston **621** has a notch **622** along the side of the piston **621.** As control piston **621** is displaced into the outlet passageway **615** by pressing on the knob **620,** the notch **622** is moved under the first sealing ring **618.** Notch **622** relives the pressure placed on the first sealing ring **618** by piston **621.** The reduction of pressure placed on the first sealing ring **618** allows gas from the inlet passageway **610** to flow around the first sealing ring **618** to the outlet passageway **615.** The profile of the notch **622** is shaped such that as the piston **621** is displaced further into the outlet passageway **615,** more gas is allowed to flow into the outlet passageway **615** from the inlet passageway **610.**

Outlet passageway **615** is in fluid communication with the endoscope passageway **616** thereby allowing gas from the gas supply to flow through shaft **12.** An endoscope, not shown, may be placed in endoscope passageway **616** and through shaft **12.** The endoscope is sized to provide sufficient gas flow between the endoscope and the inner surface of shaft **12** to separate the adventitia layer from the media layer of the artery. The endoscope is held in place by an endoscope locking mechanism **16.** Endoscope locking mechanism **16** may be a latch having a latch pad **630** that holds the endoscope in place when endoscope latch **16** is in a closed position, or, alternatively, the lcoking mechanism may be a quarter turn lock. The endoscope is sealed by pressing the endoscope against a wiper seal **633** before locking the endoscope in place. The endoscope latch **16** may be released from the closed position to an open position by a latch handle **632.** The design of the latch handle **632** is well known to one of ordinary skill in the mechanical arts. The endoscope latch **16** is moved from a closed position to an open position by rotating the endoscope latch **16** about a pivot **631.**

In one embodiment of the invention, the surgical instrument **10** may use either a disposable or reusable endoscope from a variety of manufacturers. For example, the INTRAMED® angioscope models 700070 (a 1.9 mm disposable), 702016 (1.9 mm diameter; reusable), or 702023 (2.4 mm diameter; reuseable), all from Baxter International, Inc. may be used in the present invention. Model A5000 (1.7 mm diameter; disposable angioscope) or Model A5102 (1.7 mm diameter; reusable angioscope), both from Applied Medical Resources of Laguna Hills, CA, or instruments employing similar principles, may also be used in the present invention.

In another embodiment of the invention, surgical instrument **10** may be configured without an endoscope, allowing the physician to decide if an endoscope is necessary for the particular procedure. A plug may be used instead of an endoscope to reduce the cost of the procedure. The plug is configured to form a seal with the wiper seal 633 and to be held in place by the latch pad 630. In another embodiment, the plug may also comprise of a length of plastic or metallic material having substantiality the same diameter and length of an endoscope in order to provide additional stiffness to the shaft **12.**

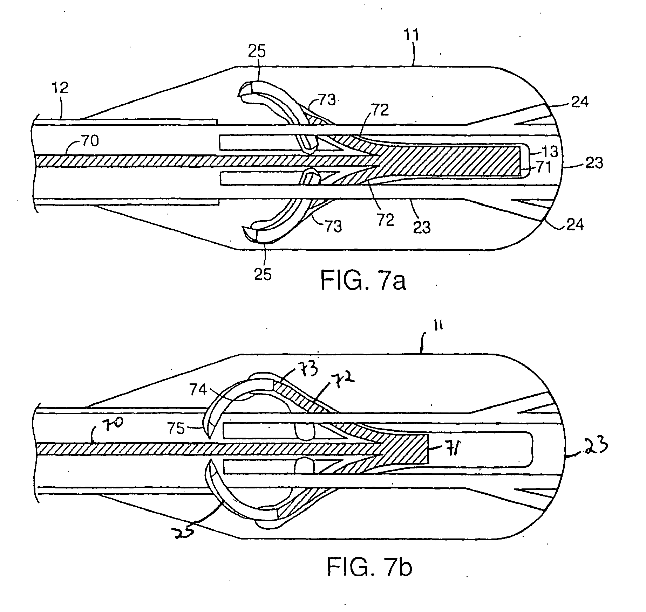

FIG. 7a shows a cut bottom view of the head of the embodiment shown in FIG. 1a showing the internal passageways within the head. Head **11 is** attached to shaft **12.** A control wire **70 is** disposed inside shaft **12** and terminates in the head **11** at the wire end **71.** Wire end **71** connects the end of the control wire 70 to the end of at least one barb wire **72.** The opposite end of the barb wire **72** is attached to the barb **25.** Control wire **70,** wire end **71,** barb wire **72,** and barb 25 are positioned in a channel **73** to allow for free movement of the wires **70, 72,** wire end **71** and barb **25** within the head **11.** The channel **73** is in fluid communication with the gas port **24** and endoscope port **23.**

FIG. 7b shows a cut bottom view of the head of the embodiment shown in FIG 1a in the deployed configuration. Deployment of the barb **25** is accomplished by withdrawing control wire **70** from head **11.** The withdrawal of control wire 70 cause wire end **71** and the barb wire **72** to move in a rearward direction away from the endoscope port **23** and toward the handle 13 (not shown). The rearward movement of the barb wire **72** pushes barb **25** along channel **73** such that barb tip 75 and barb 25 extend substantially beyond the barb channel opening **74** in the head **11.**

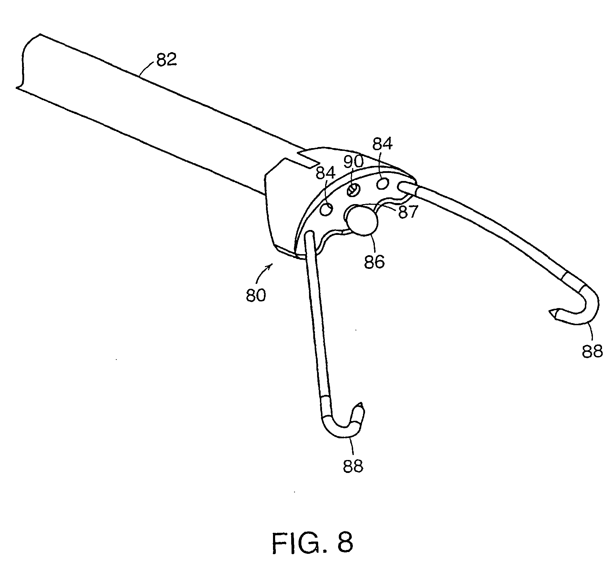

FIG. 8 shows a perspective view of head 80 of shaft 82, in accordance with other embodiments of the invention. Shaft 82 contains at least one lumen for the delivery of gas (typically carbon dioxide) and/or saline solution via one or more ports **84.** Another lumen through shaft **82** allows an endoscope to emerge at endoscope port **86.** Endoscope port **86** may be shaped, like the lumen through which the endoscope is threaded, with a notch (or 'sidechannel') **87** to provide for delivery of gas and/or saline solution through the endoscope lumen. Two retractable hooks 88 are shown, whereas a third retractable hook is preferably deployed through port 90 but has been omitted to allow clearer depiction of the central region of the head.

Saline solution is delivered for purposes of keeping the endoscope clear, and thus also the image as viewed by the surgeon. Saline solution is preferably delivered through a separate lumen rather than through an endoscope with its own working channel. A separate lumen is preferred because the working channel of an endoscope is difficult to sterilize, in that it is an internal space, and thus may require the additional expense of a disposable endoscope. An endoscope having a diameter of approximately 0.9 mm and lacking a working channel is preferably employed in this embodiment of the invention.

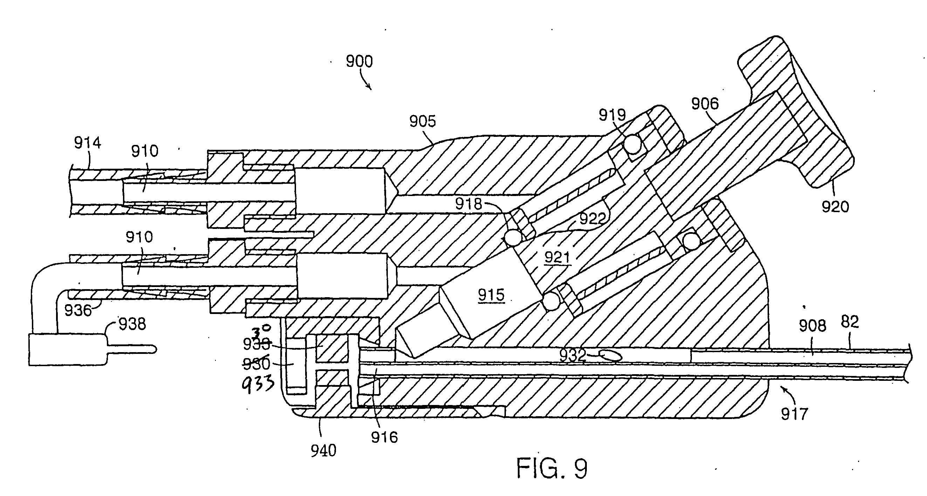

FIG. 9 is a side cut view showing the internal channels of handle 900 in the embodiment of the present invention depicted in FIG. 8. An external gas source is connected to the surgical instrument by a gas port **914** and supplies gas to the head of the surgical instrument. In a preferred embodiment, the gas is CO₂. The gas flows through the inlet passageway **910** to a variable flow valve **906,** exiting through an outlet passageway **915.** Outlet passageway **915** is connected to channel **908** which carries the gas, saline solution, and control wires through shaft **82.** Control wires are brought out of the proximal end of shaft **82** at control wire port **932.** Endoscope passageway **916** similarly carries the endoscope through shaft **82** to which it is coupled at shaft end **917.**

Variable flow valve **906** comprises a knob **920** attached to a control piston **921,** the end of which may be displaced into or out of the outlet passageway **915.** The inlet passageway **910** is isolated from the outlet passageway **915** by a first sealing ring **918.** The inlet passageway **910** is maintained in fluid isolation from the environment by a second sealing ring **919.** The first sealing ring **918** is held against the handle body **905** by the control piston **921.** Control piston **921** has a notch **922** along the side of piston **921.** As control piston **921** is displaced into the outlet passageway **915** by pressing on the knob **920,** the notch **922** is moved under the first sealing ring **918.** Notch **922** relives the pressure placed on the first sealing ring **918** by piston **921.** The reduction of pressure placed on the first sealing ring **918** allows gas from the inlet passageway **910** to flow around the first sealing ring **918** to the outlet passageway **915.** The profile of notch **922** is shaped such that as the piston **921** is displaced further into outlet passageway **915,** more gas is allowed to flow into the outlet passageway **915** from the inlet passageway **910.**

A second line **936** couples a flow of saline solution for clearing the distal end of the endoscope, as regulated, typically, by a foot pedal **938** operated by the surgeon. Saline solution is coupled, via passageway **915,** into the same channel **916** which conveys gas to the distal end of shaft **82.**

Outlet passageway **915** is in fluid communication with channel **908** through which gas, saline solution, and control wires are guided to the distal end of shaft **82.**

An endoscope (not shown, but represented by passageway **916)** may be placed in endoscope passageway **916** and through shaft **82.** The endoscope is sized to provide sufficient gas flow between the endoscope and the inner surface of shaft 82 to separate the adventitia layer from the media layer of the artery. The endoscope is held in place by an endoscope locking mechanism **940** having a latch pad 930 that holds the endoscope in place when endoscope locking mechanism **940** is in a closed position.

In an altemate embodiment of the invention, gas and saline solution may be conveyed through the same passageway **916** as contains the endoscope. In this case, a gas-tight seal **933** is preferably used.

Having thus described various illustrative embodiments of the present invention, some of its advantages and optional features, it will be apparent that such embodiments are presented by way of example only and are not by way of limitation. Those skilled in the art could readily devise alternations and improvements on these embodiments, as well as additional embodiments. For example, although a control wire is used to deploy or retract the grasping device, a collar may be attached to the endoscope end that engages the grasping device. The grasping device may be deployed or retracted by unlocking the endoscope latch on the handle and moving the endoscope forward or backward in the shaft. Alternatively, head **11** may be employed in the manner of a spatula. All such modifications are within the scope of the invention as claimed.

## Claims

1. An endarterectomy surgical instrument comprising:
(a) a shaft 12, 82 having proximal and distal ends;
(b) a head 11 coupled to the distal end of the shaft 12, 82, the head 11 having an endoscope port 23 and at least one fluid port 24; and
(c) a handle 13 coupled to the proximal end of the shaft, **characterised in that** the handle comprises:
(i) a gas supply port 14 in fluid communication with the at least one gas port on the head;
(ii) a flow valve 15 for metering flow of gas between the gas supply port and the at least one fluid port on the head;
(iii) a locking mechanism 16 for retaining an endoscope; and
further **characterised by** a grasping device 18, 43, 25 at the distal end of the shaft 12, 82 having a retracted configuration and a deployed configuration controllable from the proximal end of the shaft wherein the grasping device extends away from the head in the deployed configuration.

2. The endarterectomy surgical instrument of claim 1, further comprising a saline solution inlet 84 coupled to the handle for coupling a flow of saline solution to the at least one fluid port on the head.

3. The endarterectomy surgical instrument of claim 1, wherein a fluid connection of the handle to the head of the shaft is provided through a first lumen.

4. The endarterectomy surgical instrument of claim 1, further comprising an endoscope for providing optical coupling through a second lumen between the distal and proximal ends of the shaft 82.

5. The endarterectomy surgical instrument of claim 1, wherein a fluid connection of the handle to the head of the shaft is provided through a first lumen, further comprising an endoscope for providing optical coupling through a second lumen between the distal and proximal ends of the shaft 82.

6. The endarterectomy surgical instrument of claim 5, wherein the first lumen is identical to the second lumen.

7. The endarterectomy surgical instrument of claim 1, further comprising a deployment control 17 disposed on the handle of the instrument and in mechanical communication with the grasping device 18, 43, 25.

8. The endarterectomy surgical instrument as in claim 1, wherein the grasping device is a barb 25.

9. The endarterectomy surgical instrument as in claim 1, wherein the grasping device is a hook 43.

10. The endarterectomy surgical instrument as in claim 7, wherein the deployment control is a slide 17.

11. The endarterectomy surgical instrument of claim 7, wherein mechanical communication between the deployment control 17 and the grasping device 18, 43, 25 includes a control wire having a first wire end and a second wire end, the first wire end connected to the grasping device 18, 43, 25 and the second wire end connected to the deployment control 17.

## Patentansprüche

1. Chirurgisches Instrument zur Endarteriektomie mit:
(a) einer Welle (12, 82) mit proximalen und distalen Enden;
(b) ein Kopf (11) gekoppelt mit dem distalen Ende der Welle (12, 82), wobei der Kopf (11) eine endoskopische Öffnung (23) und wenigstens eine Öffnung (24) für ein Fluid aufweist; und
(c) eine Handhabe (13) gekoppelt mit dem proximalen Ende der Welle, **dadurch gekennzeichnet, dass** die Handhabe folgendes aufweist:
(i) einen Gaszufuhranschluss (14), wobei zwischen dem Gaszufuhranschluss (14) und wenigstens der einen Öffnung des Kopfes ein Fluidaustausch stattfindet;
(ii) einem Strömungsventil (15) zur Messung des Gasflusses zwischen dem Gaszufuhranschluss und der wenigstens einen Fluidöffung des Kopfes;
(iii) einem Verriegelungsmechanismus (16) zum Halten eines Endoskops; und
ferner **gekennzeichnet durch** eine Greifvorrichtung (18, 43, 25) an dem distalen Ende der Welle (12, 82), wobei die Greifvorrichtung eine eingefahrene Konfiguration und eine ausgefahrene Konfiguration vorsieht, die von dem proximalen Ende der Welle gesteuert werden kann, wobei die Greifvorrichtung in der ausgefahrenen Konfiguration vom Kopf wegweisend ausgefahren ist.

2. Chirurgisches Instrument zur Endarteriektomie nach Anspruch 1, das weiterhin einen Einlass (84) für eine Kochsalzlösung aufweist, welcher mit der Handhabe zur Kopplung eines Flusses von Kochsalzlösung zu der wenigstens einer Fluidöffnung des Kopfes gekoppelt ist.

3. Chirurgisches Instrument zur Endarteriektomie nach Anspruch 1, wobei eine Fluidverbindung der Handhabe zu dem Kopf der Welle durch ein erstes Lumen vorgesehen ist.

4. Chirurgisches Instrument zur Endarteriektomie nach Anspruch 1, welches ferner ein Endoskop zur Bereitstellung einer optischen Kupplung durch ein zweites Lumen zwischen dem distalen und dem proximalen Ende der Welle (82) aufweist.

5. Chirurgisches Instrument zur Endarteriektomie nach Anspruch 1, wobei eine Fluidverbindung des Griffs zu dem Kopf der Welle durch ein erstes Lumen vorgesehen ist, ferner ein Endoskop zur Bereitstellung einer optischen Kupplung durch ein zweites Lumen zwischen dem distalen und dem proximalen Ende der Welle (82) vorsieht.

6. Chirurgisches Instrument zur Endarteriektomie nach Anspruch 5, wobei das erste Lumen identisch zu dem zweiten Lumen ist.

7. Chirurgisches Instrument zur Endarteriektomie nach Anspruch 1, welches weiterhin eine auf der Handhabe des Instruments angeordnete Ausfahrsteuerung (17), welche mit der Greifvorrichtung (18, 43, 25) mechanisch gekoppelt ist, vorsieht.

8. Chirurgisches Instrument zur Endarteriektomie nach Anspruch 1, wobei die Greifvorrichtung als Widerhaken (25) ausgebildet ist.

9. Chirurgisches Instrument zur Endarteriektomie nach Anspruch 1, wobei die Greifvorrichtung als Haken (43) ausgebildet ist.

10. Chirurgisches Instrument zur Endarteriektomie nach Anspruch 7, wobei die Ausfahrsteuerung als Schieber (17) ausgebildet ist.

11. Chirurgisches Instrument zur Endarteriektomie nach Anspruch 7, wobei eine mechanische Kopplung zwischen der Ausfahrsteuerung (17) und der Greifvorrichtung (18, 43, 25) einen Steuerdraht mit einem ersten Drahtende und einen zweiten Drahtende aufweist, wobei das erste Drahtende mit der Greifvorrichtung (18, 43, 25) und das zweite Drahtende mit der Ausfahrsteuerung (17) verbunden ist.

## Revendications

1. Instrument chirurgical d'endartérectomie comprenant :
(a) une tige 12, 82 ayant des extrémités proximale et distale ;
(b) une tête 11 couplée à l'extrémité distale de la tige 12, 82, la tête 11 comportant un orifice d'endoscope 23 et au moins un orifice de fluide 24, et
(c) une poignée 13 couplée à l'extrémité proximale de la tige, **caractérisé en ce que** la poignée comprend :
(i) un orifice d'alimentation en gaz 14 en communication de fluide avec le au moins un orifice de gaz situé sur la tête ;
(ii) une soupape d'écoulement 15 destinée à mesurer l'écoulement de gaz entre l'orifice d'alimentation en gaz et le au moins un orifice de fluide situé sur la tête ;
(iii) un mécanisme de verrouillage 16 destiné à retenir un endoscope ; et
**caractérisé en outre par** un dispositif de préhension 18, 43, 25 au niveau de l'extrémité distale de la tige 12, 82 ayant une configuration rétractée et une configuration déployée pouvant être commandées depuis l'extrémité proximale de la tige, dans lequel le dispositif de préhension s'étend en s'éloignant de la tête dans la configuration déployée.

2. Instrument chirurgical d'endartérectomie selon la revendication 1, comprenant en outre une entrée de solution saline 84 couplée à la poignée pour coupler un écoulement de solution saline au au moins un orifice de fluide situé sur la tête.

3. Instrument chirurgical d'endartérectomie selon la revendication 1, dans lequel un raccordement de fluide de la poignée à la tête de la tige est établi par le biais d'une première lumière.

4. Instrument chirurgical d'endartérectomie selon la revendication 1, comprenant en outre un endoscope destiné à établir un couplage optique par le biais d'une deuxième lumière entre les extrémités distale et proximale de la tige 82.

5. Instrument chirurgical d'endartérectomie selon la revendication 1, dans lequel un raccordement de fluide de la poignée à la tête de la tige est établi par le biais d'une première lumière, comprenant en outre un endoscope destiné à établir un couplage optique par le biais d'une deuxième lumière entre les extrémités distale et proximale de la tige 82.

6. Instrument chirurgical d'endartérectomie selon la revendication 5, dans lequel la première lumière est identique à la deuxième lumière.

7. Instrument chirurgical d'endartérectomie selon la revendication 1, comprenant en outre un dispositif de commande de déploiement 17 disposé sur la poignée de l'instrument et en communication mécanique avec le dispositif de préhension 18, 43, 25.

8. Instrument chirurgical d'endartérectomie selon la revendication 1, dans lequel le dispositif de préhension est un ardillon 25.

9. Instrument chirurgical d'endartérectomie selon la revendication 1, dans lequel le dispositif de préhension est un crochet 43.

10. Instrument chirurgical d'endartérectomie selon la revendication 7, dans lequel le dispositif de commande de déploiement est une glissière 17.

11. Instrument chirurgical d'endartérectomie selon la revendication 7, dans lequel la communication mécanique entre le dispositif de commande de déploiement 17 et le dispositif de préhension 18, 43, 25 comprend un fil de commande ayant une première extrémité de fil et une seconde extrémité de fil, la première extrémité de fil étant reliée au dispositif de préhension 18, 43, 25 et la seconde extrémité de fil étant reliée au dispositif de commande de déploiement 17.
